# EUROPEAN PATENT APPLICATION

(11) **EP 0 526 868 A2**
(43) Date of publication of application: **10.02.1993**
(21) Application number: 92113222.1
(22) Date of filing: 03.08.1992
(51) Int. Cl.: A61F 13/15

(54) **Disposable absorbent pant**

(30) Priority: 07.08.1991 US 741277
(71) Applicant: KIMBERLY-CLARK CORPORATION, Neenah, Wisconsin 54956-0349 (US)
(72) Inventor: Suprise, Jody Dorothy, Neenah, Wisconsin 54956 (US); Van Gompel, Paul Theodore, Hortonville, Wisconsin 54944 (US); Popp, Robert Lee, Hortonville, Wisconsin 54944 (US)
(74) Representative: Diehl, Hermann O. Th., Dr.

(57) **Abstract**

A disposable absorbent child's training pant (2) having stretchable panels (10) that assist in easily fitting the training pant (2) onto a child and snugly maintaining it in place both before and after an insult. Full waist and leg elastics (24, 26) are provided to improve the fit, performance, and appearance of the pant.

## Description

This invention pertains to a disposable absorbent pant.

Currently, disposable absorbent garments are widely used and generally comprise a three-layered structure having a liquid-permeable topsheet, a liquid-impermeable backsheet, and some type of absorbent between the topsheet and backsheet. In order to improve the fit and function of the disposable garment, various elastic structures have been incorporated, such as waist elastics and leg elastics.

Although these current structures are widely used by the public, there is still need of improvement in certain areas, such as a closer-to-the-body fit, better containment of body wastes, more comfortable fit, and the like.

This object is solved by the disposable absorbent pant according to independent claim 1 or 11. Further advantageous features, aspects and details are evident from the dependent claims, the description and the drawings. The claims are intended to be understood as a first non-limiting attempt of defining the invention in general terms.

The invention provides particularly a disposable absorbent pant having stretchable panels for use as a child's training pant.

In one form of the invention there is provided a disposable absorbent pant comprising a generally T-shaped backsheet including a non-stretchable central strip having front and back end portions and a pair of non-stretchable ear portions disposed on respective opposite sides of the back end portion. An absorbent medium is disposed on at least the central strip, and a pair of stretchable ear portions are disposed on respective opposite sides of the front end portion. The non-stretchable ear portions are joined with the stretchable ear portions so as to form together with the front and back end portions a waist opening and a pair of leg openings.

In another form of the present invention there is provided a disposable absorbent training pant comprising a main body having a front panel, a back panel, a crotch panel, and a pair of side panels forming a waist opening and a pair of leg openings. A stretch-bonded laminate waistband and stretch-bonded laminate leg members are disposed at the waist opening and leg openings, respectively.

The above-mentioned and other features and objects of this invention and the manner of attaining them will become more apparent, and the invention itself will be better understood by reference to the following description of the invention, taken in conjunction with the accompanying drawings, wherein:
Fig. 1 is a top plan view of a modification of the preferred embodiment of Fig. 5 in a flat, unassembled state;
Fig. 2 is a front elevational view of the modification in Fig. 1 assembled with a waist opening and a pair of leg openings;
Fig. 3 is a top plan view of another modification in a flat, unassembled state;
Fig. 4 is a front elevational view of the modification in Fig. 3 assembled with a waist opening and a pair of leg openings;
Fig. 5 is a top plan view of a preferred embodiment of the present invention in a flat, unassembled state;
Fig. 6 is a front elevational view of the embodiment in Fig. 5 assembled with a waist opening and a pair of leg openings;
Figs. 7-8 are cross-sections through different absorbent panels; and
Figs. 9-14 are cross-sections showing various structures and placements of stretchable members in the present invention.

### Definitions

Within the context of this specification, each term below will include the following meaning:
(a) "Disposed", "disposed on", "disposed with", "disposed at", and variations thereof, are intended to mean that one element can be integral with another element, or that one element can be a separate structure joined to or placed with or placed near another element.
(b) "SBL" or "stretch-bonded laminate" means at least a two-layered composite in which one layer is a gatherable layer and the other layer a stretchable layer. The layers are joined together when the stretchable layer is in a stretched condition so that upon relaxing the layers, the gatherable layer is gathered.
(c) "Particles" as used in superabsorbent particles means any geometric form such as, but not limited to, spherical grains, cylindrical fibers or strands, flat surfaces or roughened surfaces, sheets, ribbons, strings, strands, or the like.

These definitions are not intended to be limiting, and these terms may be defined with additional language in the remaining portion of the specification.

### Detailed Description

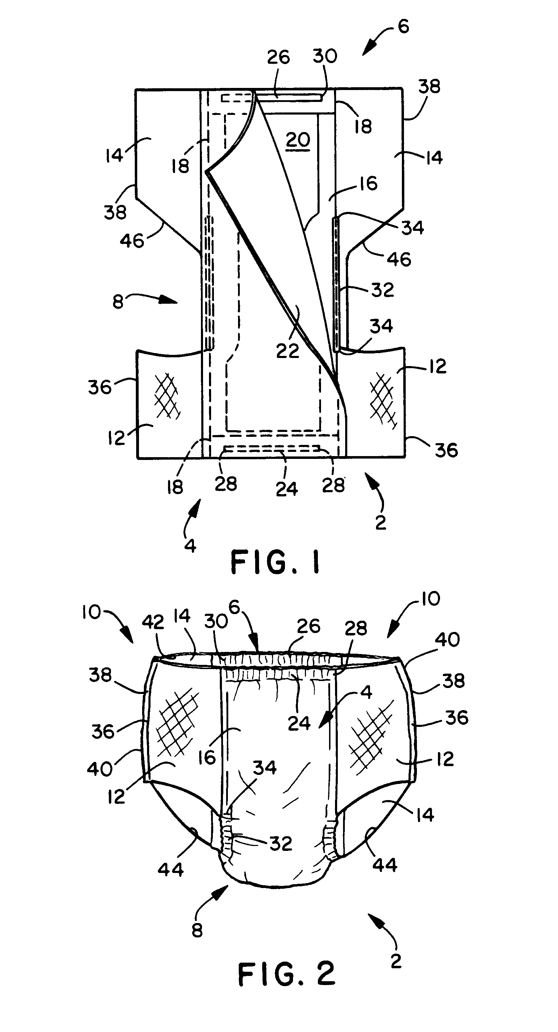

Referring primarily to Figs. 1 and 2, disposable absorbent article 2 of the present invention comprises front central panel 4, back central panel 6, and absorbent crotch panel 8 disposed between front central panel 4 and back central panel 6. Article 2 further includes a pair of side panels 10 (Fig. 2) wherein each side panel 10 comprises a stretchable front ear portion or front section 12 and a non-stretchable back ear portion or back section 14. Panels 4, 6, 8, include backsheet 16, which is generally rectangular in shape, having opposite lateral sides 18 that extend the length of panels 4, 6, 8. Although panels 4, 6, 8 can be three separate panels joined together by any suitable means, it is preferred that they are integral with one another.

Disposable article 2 further comprises absorbent medium 20 and, if desired, liquid-permeable topsheet 22. Fig. 1 illustrates absorbent medium 20 as having a generally hour-glass shape and extending generally the length of absorbent article 2. However, the present invention contemplates that only crotch panel 8 can be absorbent, while front and back central panels 4, 6 are relatively non- absorbent. In the present description, absorbent medium 20 is described and illustrated as extending from crotch panel 8 into at least a portion of central panels 4, 6. Topsheet 22 is described and illustrated as extending the general length of absorbent article 2 from front central panel 4 to back central panel 6. Depending upon the material composition of absorbent medium 20, topsheet 22 may or may not be necessary.

Although front section 12 of each side panel 10 has been described as stretchable, the present invention contemplates that back section 14, rather than front section 12, can be stretchable. The section 12 or 14 of a respective side panel 10 that is non-stretchable also can be integral with backsheet 16. Thus, with reference to Fig. 1, backsheet 16 can be an integral T-shaped liquid-impermeable backsheet.

Stretchable front waistband 24 is disposed with front central panel 4, and stretchable back waistband 26 is disposed with back central panel 6. Each waistband 24, 26 is illustrated in Fig. 1 as being between backsheet 16 and topsheet 22. Other configurations for waistbands 24, 26 will be described hereafter. Stretchable front waistband 24 includes opposite ends 28, and stretchable back waistband 26 includes opposite ends 30. In Fig. 1, waistbands 24, 26 extend not quite the full distance between lateral sides 18 of backsheet 16.

A pair of stretchable leg elastics or members 32 are disposed on either side of absorbent crotch panel 8 and generally between backsheet 16 and topsheet 22. Each leg elastic 32 includes opposite ends 34.

Each front section 12 includes outermost side 36, and each back section 14 includes outermost side 38. By joining a side 36 to a side 38 at a seam 40 (Fig. 2), there is formed waist opening 42 and a pair of leg openings 44. Seams 40 can be formed in any suitable manner, such as by ultrasonic bonding, thermal bonding, adhesive bonding, and the like. For ease of manufacture, each side panel 10 has been described as having two components, front and back sections 12, 14. However, the present invention contemplates that each side panel 10 can be integral, i.e., without separate front and back sections 12,14, in which its opposite sides are joined to lateral sides 18 to form waist opening 42 and leg openings 44. Regardless of the means of manufacturing disposable absorbent article 2, it is important that one of the sections 12, 14 be stretchable.

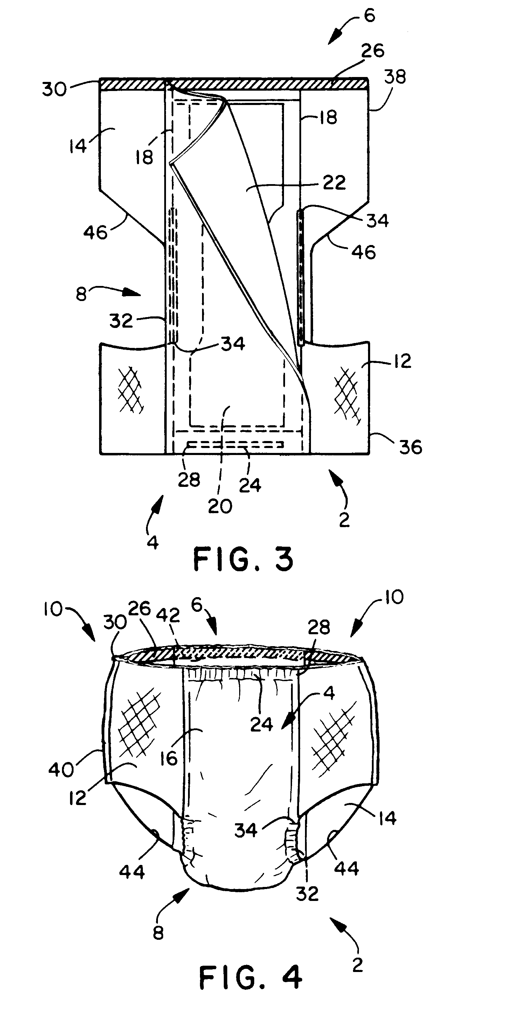

Referring now to Figs. 3 and 4, there is another modification in which again each front section 12 of a side panel 10 is stretchable and each back section 14 is non-stretchable relative to front section 12. Stretchable back waistband 26 extends the distance, as viewed or measured horizontally in Fig. 3, between side 38 of one back section 14 and across back central panel 6 to side 38 of the other back section 14. When front and back sections 12, 14 are joined to form seams 40, ends 30 of stretchable back waistband 26 are adjacent respective stretchable front sections 12. Ends 28 of stretchable front waistband 24 also are adjacent stretchable front section 12. It is preferred that ends 28, 30 touch or overlap with stretchable front sections 12 so as to provide full peripheral elasticity to waist opening 42. By providing full front and back waistbands 24, 26, with stretchable front sections 12, waist opening 42 is stretchable about its periphery. This provides several benefits, such as improving back waist fit, improving the overall appearance of the article on the wearer, and providing a reinforcement band or point which the wearer may grasp in pulling up or pulling down article 2.

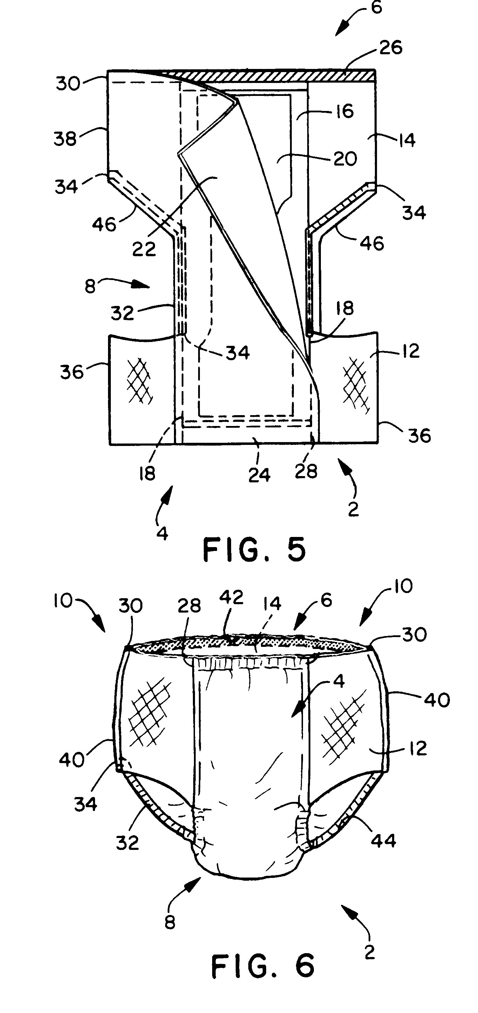

Referring now to Figs. 5 and 6, a preferred embodiment of the present invention is illustrated in which each stretchable leg member 32 is extended along a respective inner side 46 of non-stretchable back section 14 such that an end 34 of leg member 32 is adjacent side 38 of back section 14. Thus, upon joining front and back sections 12, 14 as described above, ends 34 of each leg elastic or member 32 is adjacent a stretchable front section 12, thereby providing stretchability about the periphery of each leg opening 44. This stretchability about each leg opening 44 has several advantages, such as improving leg fit, improving BM and urine containment, and improving the appearance of article 2 on the wearer. As illustrated in Fig. 6, this embodiment of the present invention provides stretchability about the peripheries of waist opening 42 and leg openings 44.

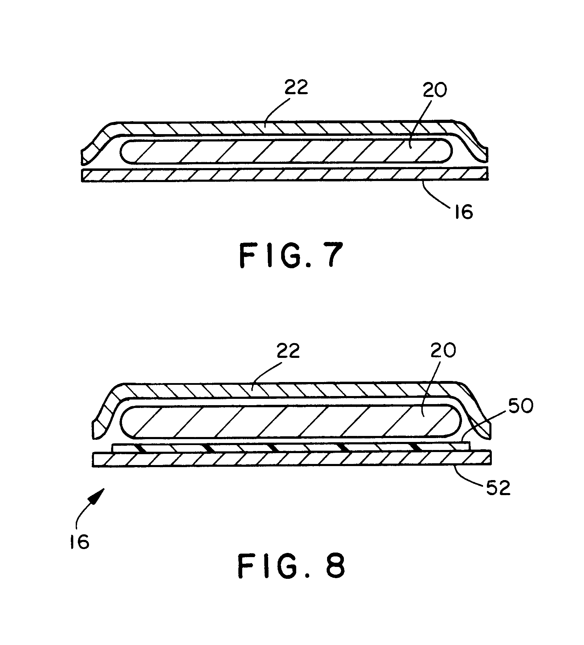

Referring to Figs. 1-8, absorbent areas of disposable absorbent article 2 may include singly or in any combination front central panel 4, back central panel 6, crotch panel 8, and the non-stretchable section 12 or 14. Regardless of the geometric shape or area of absorbency, its structure can include, as illustrated in Fig. 7, liquid-permeable topsheet 22 and liquid-impermeable backsheet 16, sandwiching therebetween absorbent medium 20. If it is preferred to give backsheet 16 a cloth-like appearance and feel, then backsheet 16 can be a two-ply backsheet comprising (Fig. 8) liquid-impervious film 50 and a nonwoven layer 52, in which film 50 and layer 52 may or may not be joined together.

Backsheet 16 may be made of any suitable material that is liquid impermeable. Examples include meltblown or film material made of polypropylene or polyolefin, copolymers such as ethylene vinyl acetate, ethylene methyl acrylate, ethylene ethyl acrylate, polyvinyl chloride, and the like. Other materials include a single spunbonded layer of the above types of material, two layers of spunbonded and meltblown materials, or three layers of material of spunbonded-meltblown-spunbonded material; each of which is suitably treated or coated to be liquid impermeable. Backsheet 16 may also be made of a material that is liquid impermeable, and vapor permeable, thereby providing a breathability feature to the article.

Topsheet 22 may be a liquid permeable, hydrophilic or hydrophobic material, such as a spunbonded web composed of synthetic polymer filaments; a spunlace web; a spunbond-meltblown web; a meltblown web; or a bonded carded web composed of synthetic polymer fibers. Suitable synthetic polymers include polyethylene, polypropylene, polyester, and nylon.

Absorbent layer or medium 20 may be made of any suitable absorbent materials such as cellulosic fibers, synthetic fibers, absorbent gelling materials in the form of particles, fibers, layers, and the like. These various materials may be combined as mixtures or blends. They also may be discrete layers such as a discrete layer of cellulosic fiber and a discrete layer of absorbent gelling materials, or a coform-type layer which is a blend or mixture of synthetic and cellulosic fibers formed as a coform layer with a discrete layer of absorbent gelling materials placed therewith, or a discrete layer comprising a mixture or blend of absorbent gelling materials with cellulosic or synthetic fibers. Any number of combinations is possible, and the present invention contemplates mixtures, layers that may include one or more absorbent materials mixed together, or various combinations of mixtures and discrete layers. Suitable absorbent gelling materials can be inorganic materials such as silica gels or organic compounds such as crosslinked polymers. Examples include polyacrylamides, polyvinyl alcohol, polyacrylates, acrylonitrile grafted starch, acrylic acid grafted starch, modified carboxymethylcellulose and the like. Absorbent medium or layer 20 can also include a tissue wrap to maintain the integrity thereof.

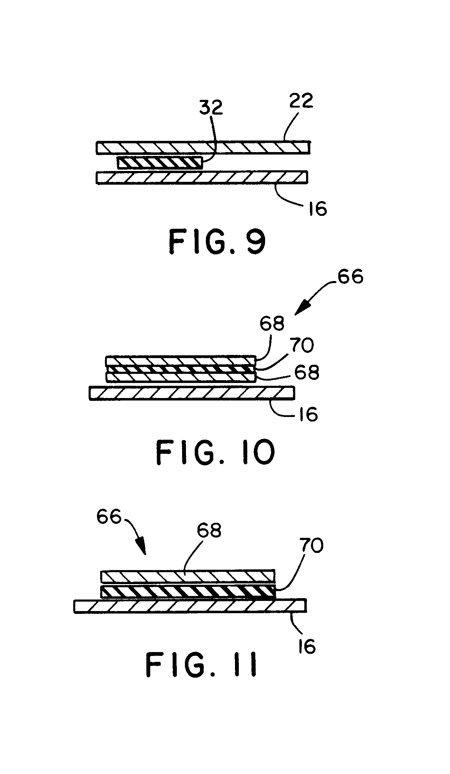

Referring to Fig. 9, there is illustrated a cross section through a portion of crotch panel 8 illustrating the relative placement of topsheet 22, backsheet 16 and leg elastic 32. Generally, leg elastic 32 is first stretched and then applied to either one or both of backsheet 16 and topsheet 22, and upon relaxing leg elastic or member 32, that portion of crotch panel 8 is gathered. Leg members 32 can also be heat-shrinkable material in which they would be applied in an unstretched manner between topsheet 22 and backsheet 16 and then with the application of heat, leg members 32 will contract to gather crotch panel 8.

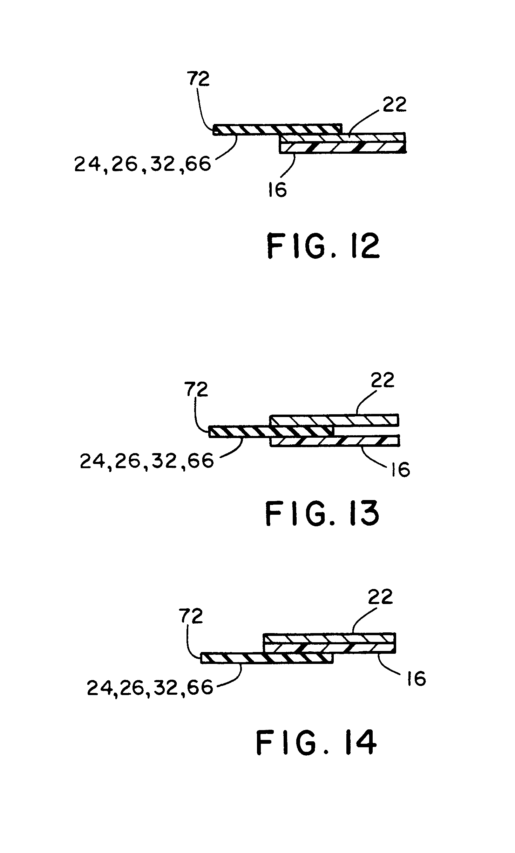

Referring to Fig. 10, there is illustrated a stretch-bonded laminate 66 comprising nonwoven layers 68 sandwiching elastomeric layer 70. Stretch-bonded laminate (SBL) 66 is then applied or attached to backsheet 16. Alternatively, Fig. 11 illustrates SBL 66 with a single nonwoven layer 68, and elastomeric layer 70 applied to backsheet 16. In this embodiment, elastomeric layer 70 is a heat-shrinkable material which after having been attached or applied to both nonwoven layer 68 and backsheet 16 has heat applied thereto in order to gather or contract nonwoven layer 68 and that portion of backsheet 16 to which it is attached to provide stretchability thereto. Waistbands 24,26 can be provided in a manner similar to leg members 32.

Figs. 12-14 illustrate the various ways that waistbands 24, 26 and leg members 32 can be attached or applied to absorbent article 2. Waistbands 24, 26 and leg members 32 can be a single layer of elastomeric film or a stretch-bonded laminate, such as SBL 66. Regardless of the structure of waistbands 24, 26 and leg members 32, they may be attached in various ways, some of which are illustrated in Figs. 12-14. In Fig. 12, waistbands 24, 26 or leg members 32 are attached to the inner surface of topsheet 22 such that their outermost edge 72 extends laterally, outwardly beyond topsheet 22 and backsheet 16. In Fig. 13, waistbands 24, 26 or leg members 32 are attached between topsheet 22 and backsheet 16, with edge 72 extending laterally outwardly beyond both topsheet 22 and backsheet 16. In Fig. 14, waistbands 24, 26 or leg members 32 are attached to the outer surface of backsheet 16 with edge 72 of a respective waistband 24, 26 or leg member 32 extending laterally and outwardly beyond topsheet 22 and backsheet 16.

Waistbands 24, 26 and leg members 32 can be a SBL, a single ribbon of material, or a plurality of strings or ropes of stretchable material. Waistbands 24, 26, leg members 32, and stretchable front or back sections 12, 14 can be made of any suitable elastic materials, such as natural rubber, synthetic rubber, or thermoplastic elastomeric polymers. These materials may also be heat-shrinkable or heat-elasticizable.

Elastomeric layer 70 of SBL 66 can also be made of the above types of stretchable or elastic materials, and nonwoven layer 68 can be made of any suitable type material.

## Claims

1. A disposable absorbent pant (2), comprising:
a non-stretchable T-shaped backsheet (16) comprising a non-stretchable central strip having non-stretchable front and back end portions, and a pair of non-stretchable back ear portions (14) disposed on respective opposite sides of said non-stretchable back end portion, thereby forming the T-shape of said backsheet (16),
an absorbent medium (20) disposed on at least said non-stretchable central strip, and
a pair of stretchable front ear portions (12) disposed on respective opposite sides of said non-stretchable front end portion,
said non-stretchable back ear portions (14) being joined with said stretchable front ear portions (12) to form a waist opening (42) and a pair of leg openings (44).

2. The pant of claim 1 further comprising a stretchable back waistband (26) extending along said back end portion ( 6) of said non-stretchable central strip and said non-stretchable back ear portions (14), said stretchable back waistband (26) having opposite ends (30) terminating at respective ones of said stretchable front ear portions (12).

3. The pant of claim 1 or 2 further comprising a stretchable front waistband (24) extending along said front end portion (4) of said non-stretchable central strip, said stretchable front waistband (24) having opposite ends (28) terminating at respective ones of said stretchable front ear portions (12).

4. The pant of any one of the preceding claims further comprising a stretchable leg member (32) disposed at least at a crotch portion of each said leg opening (44).

5. The pant of claim 4 wherein each said stretchable leg member (32) has opposite end portions (34), said end portions (34) terminating at respective ones of said stretchable front ear portions (12).

6. The pant of any one of the claims 2 to 5 wherein said front waistband (24), said back waistband (26), and said leg members (32) are stretch-bonded laminates (66).

7. The pant of claim 6 wherein said stretch-bonded laminates (66) are disposed on an outer surface of said backsheet (16).

8. The pant of claim 6 wherein said stretch-bonded laminates (66) are disposed on an inner surface of said backsheet (16).

9. The pant of claim 6 further comprising a topsheet (22) disposed with said absorbent medium (20) on a side thereof opposite from said backsheet (16), and
wherein said stretch-bonded laminates (66) are disposed on a surface of said topsheet (22).

10. The pant of any one of claims 4 to 9 wherein an edge portion (72) of each said stretchable leg member (32) extends laterally, outwardly beyond said backsheet (16).

11. A disposable child's training pant (2) especially according to claim 1, comprising:
a main body comprising a front panel (4), a back panel (6), a crotch panel (8), and a pair of side panels (10) forming a waist opening (42) and a pair of leg openings (44),
a stretch-bonded laminate waistband (24, 26) comprising a stretchable layer and a gatherable layer, and being disposed at said waist opening (42), and
a stretch-bonded laminate leg member (32) comprising a stretchable layer and a gatherable layer, and being disposed at a respective said leg opening (44),
said stretch-bonded laminate leg member (32) extending laterally, outwardly beyond its respective said leg opening (44).

12. The pant of claim 11 wherein said leg members (32) are on an outer surface of said main body.

13. The pant of claim 11 wherein said leg members (32) are on an inner surface of said main body.

14. The pant of any one of claims 11 to 13 wherein said waistband (24, 26) is on an outer surface of said main body.

15. The pant of any one of claims 11 to 13 wherein said waistband (24, 26) extends laterally, outwardly beyond said waist opening (42).

16. The pant of any one of claims 11 to 15 wherein said main body further comprises a liquid impermeable backsheet (16), a liquid permeable topsheet (22), and an absorbent medium therebetween (20).

17. The pant of claim 16 wherein said waistband (24, 26) and said leg members (32) are between said backsheet (16) and said topsheet (22).

18. The pant of claim 16 wherein said waistband (24, 26) and said leg members (32) are on an inner surface of said topsheet (22).

19. The pant of claim 16 wherein said waistband (24, 26) and said leg members (32) are on an outer surface of said backsheet (18).

20. The pant of claim 17, 18, or 19 wherein said waistband (24, 26) extends laterally, outwardly beyond said waist opening (42).
